(19) Europäisches Patentamt
European Patent Office
Office européen des brevets

(11) Publication number: **0 423 714 B1**

(12) # EUROPEAN PATENT SPECIFICATION

(45) Date of publication of patent specification: **22.06.94**

(51) Int. Cl.5: **A61K 7/06**

(21) Application number: **90119814.3**

(22) Date of filing: **16.10.90**

(54) **Hair revitalizing agent.**

(30) Priority: **16.10.89 JP 266106/89**

(43) Date of publication of application:
**24.04.91 Bulletin 91/17**

(45) Publication of the grant of the patent:
**22.06.94 Bulletin 94/25**

(84) Designated Contracting States:
**AT BE CH DE DK ES FR GB GR IT LI LU NL SE**

(56) References cited:
**EP-A- 0 315 978**
**EP-A- 0 323 042**
**EP-A- 0 356 399**

(73) Proprietor: **FUJISAWA PHARMACEUTICAL CO., LTD.**
**4-7, Doshomachi 3-chome**
**Chuo-ku**
**Osaka-shi Osaka 541(JP)**

(72) Inventor: **Honbo, Toshiyasu**
**3-2-1-61-904, Minatojimanakamachi,**
**Chuo-ku**
**Kobe-shi, Hyogo(JP)**
Inventor: **Hata, Takehisa**
**2-4-2, Kayougaoka**
**Nagaokakyo-shi, Kyoto(JP)**
Inventor: **Ishino, Akihiro**
**Shiseido Nippa-ryo, 338, Nippa-cho,**
**Kohoku-ku**
**Yokohama-shi, Kanagawa(JP)**
Inventor: **Tsuji, Yoshiharu**
**Shiseido Nippa-ryo, 338, Nippa-cho,**
**Kohoku-ku**
**Yokohama-shi, Kanagawa(JP)**

(74) Representative: **Türk, Gille, Hrabal, Leifert**
**Brucknerstrasse 20**
**D-40593 Düsseldorf (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

Background of the invention

1. Field of the Invention

This invention relates to the use of a tricyclo compound represented by formula (I) shown below or a pharmaceutically acceptable salt thereof for the manufacture of a medicament for treating male pattern alopecia or alopecia senilis:

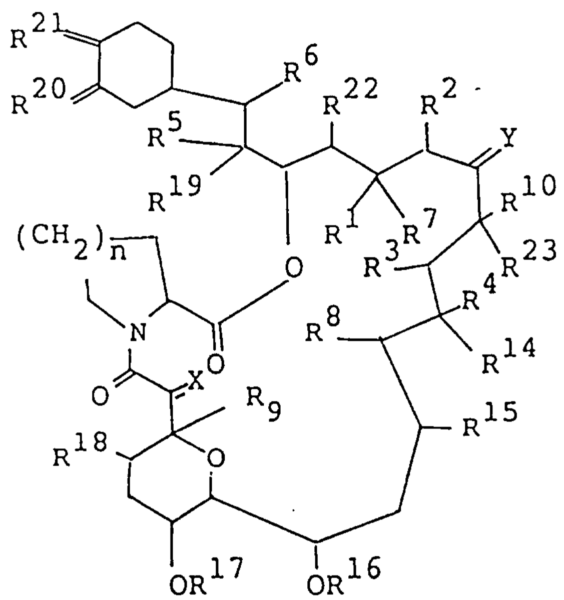

(I)

In the above formula, each adjoining pair of $R^1$ and $R^2$, $R^3$ and $R^4$, $R^5$ and $R^6$ independently,

a) represents two adjoining hydrogen atoms, or

b) forms another bond with a carbon atom to which it is bonded, and in addition thereto, $R^2$ may be an alkyl group;

$R^7$ represents a hydrogen atom, a hydroxy group, a protected hydroxy group, or an alkyloxy group, or an oxo group together with $R^1$;

$R^8$ and $R^9$ independently represent a hydrogen atom or hydroxy group;

$R^{10}$ represents a hydrogen atom, an alkyl group, an alkyl group substituted with 1 or more hydroxy group, an alkenyl group, an alkenyl group substituted with 1 or more hydroxy group, or an alkyl group substituted with an oxo group;

X represents an oxo group, (hydrogen atom, hydroxy group), (hydrogen atom, hydrogen atom) or a group represented by $-CH_2O-$;

Y represents an oxo group, (hydrogen atom, hydroxy group), (hydrogen atom, hydrogen atom), or a group represented by the formula $N-NR^{11}R^{12}$ or $N-OR^{13}$;

$R^{11}$ and $R^{12}$ independently represent a hydrogen atom, an alkyl group, an aryl group or a tosyl group;

$R^{13}$, $R^{14}$, $R^{15}$, $R^{16}$, $R^{17}$, $R^{18}$, $R^{19}$, $R^{22}$ and $R^{23}$ independently represent a hydrogen atom or an alkyl group;

$R^{20}$ and $R^{21}$ independently represent an oxo group, or each independently ($R^{20}_a$ , hydrogen atom) and ($R^{21}_a$ , hydrogen atom), $R^{20}_a$ and $R^{21}_a$ independently represent an hydroxy group, an alkyloxy group or a group represented by the formula $OCH_2OCH_2CH_2OCH_3$ , or $R^{21}_a$ represents a protected a hydroxy group, and further, $R^{20}_a$ and $R^{21}_a$ taken together form an oxygen atom in an epoxy ring, and

n represents 1, 2 or 3.

In addition to the above, Y and $R^{10}$ and $R^{23}$, together with the carbon atoms to which they are bonded, represent a heterocyclic group containing a nitrogen atom, sulfur atom or oxygen atom comprising a saturated or unsaturated 5- or 6-membered ring, and the heterocyclic group may be substituted with one or more group selected from an alkyl group, a hydroxy group, an alkyl group substituted with one or more

hydroxy groups, an alkyloxy group, a benzyl group, and a group represented by $-CH_2Se(C_6H_5)$.

2. Description of the prior art

Tricyclo compounds of the above formula (I) and pharmaceutically acceptable salts thereof are already known to have pharmacological effects such as an excellent immunosupressive action and antimicrobial actions.

According to JP-A-61-148 181 such compounds are useful for the therapy and prophylaxis of a rejection of transplanted organs or tissues, graft-versus-host diseases reactions to grafting, various autoimmune diseases, and infectious diseases.

According to EP-A-0 315 978 such compounds, p.e. compound FK 506 as defined below, can be used for the treatment of alopecia areata among others.

Baldness or alopecia, in addition to (1) male pattern alopecia and (2) alopecia senilis, includes (3) alopecia areata, and further, diseases accompanied by basic skin lesions such as (4) cicatrix or (5) infectious tumors, or (6) accompanied by systemic disorders, for example, an internal secretion abnormality or nutritional disorder.

Among the above, (3) to (6) can all heal naturally or can be healed by curing the causes thereof.

Also, concerning (3) alopecia areata, it is considered that an autoimmune phenomenon participates therein, and therefore, the administration of a substance having an immunosuppressive action has been attempted. Accordingly, the tricyclo compound (I), which has a potent immunosuppressive action, obviously can have a therapeutical effect on alopecia areata.

On the other hand, the causes of (1) male pattern alopecia and (2) alopecia senilis are considered to be (a) an activation of male hormones at organs such as hair roots and the sebum gland, (b) a lowering in the amount of blood reaching the hair follicles, (c) a scalp abnormality caused by an excessive secretion of sebum, a formation of peroxides, or a propagation of bacteria, (d) genetic causes, and (e) aging.

For such reasons, hair revitalizing materials of the prior art generally comprise compounds having the actions of removing or alleviating the causes mentioned above formulated therein. For example, a compound having the action of inhibiting the activation of male hormones, or a compound having the action of increasing the amount of blood reaching the hair follicles, is formulated.

Nevertheless, in male pattern alopecia and alopecia senilis, the epilation mechanism and the hair generation mechanism are very complicated, and by merely inhibiting an activation of male hormones or increasing the amount of blood reaching the hair follicles, as practiced in the prior art, does not sufficiently treat or prevent baldness or alopecia.

Accordingly, the object of the present invention is the development of a medicament for treating male pattern alopecia and alopecia senilis, by which a satisfactory effect is exhibited. Therefore, the present inventors made an intensively study of this problem, and consequently, unexpectedly found that the tricyclo compound (I) shown above has an excellent hair revitalizing effect for male pattern alopecia and alopecia senilis, to thereby accomplish the present invention.

## Summary of the Invention

According to the present invention, there is provided a new use of a substance which has been newly found to have an excellent hair revitalizing effect. More specifically, the present invention provides a new use of a compound represented by the formula (I) shown below or a pharmaceutically acceptable salt thereof for the manufacture of a medicament for treating male pattern alopecia or alopecia senilis:

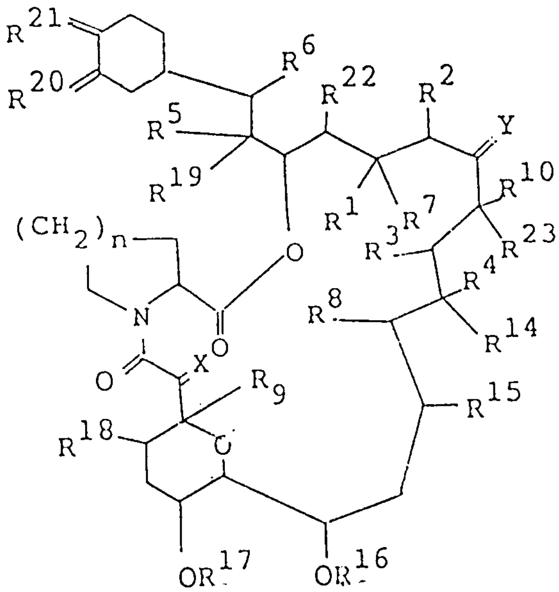

(I)

Wherein, $R^1$ to $R^{10}$, $R^{14}$ to $R^{23}$, X, Y, and n are as defined above.

According to a preferred embodiment of the present invention the compound FK-506 as defined below is used to combat male pattern alopecia or alopecia senilis.

Description of the preferred embodiments

The present invention is now described in detail.

In the present specification, "lower" means a group having 1 to 6 carbon atoms, unless otherwise specified.

Preferable examples of the "alkyl group" include straight or branched aliphatic hydrocarbon residues, for example, lower alkyl groups such as methyl, ethyl, propyl, isopropyl, butyl, isobutyl, pentyl, neopentyl, and hexyl.

Preferable examples of the "alkenyl group" include straight or branched aliphatic hydrocarbon residues containing one double bond, for example, lower alkenyl groups such as vinyl, propenyl, butenyl, methylpropenyl, pentenyl and hexenyl.

Preferable examples of the "aryl group" include phenyl, tolyl, xylyl, cumenyl, mesityl, and naphthyl.

As suitable "protective groups" in the protected hydroxy group, there are included, for example, 1-(lower alkylthio) (lower) alkyl groups such as methylthiomethyl, ethylthiomethyl, propylthiomethyl, isopropylthiomethyl, butylthiomethyl, isobutylthiomethyl, and hexylthiomethyl, more preferably $C_1$-$C_4$ alkylthiomethyl group, most preferably methylthiomethyl group; tri-substituted silyl groups, for example, tri-(lower)alkylsilyl such as trimethylsilyl, triethylsilyl, tributylsilyl, tert-butyl-dimethylsilyl, and tri-tert-butylsilyl, lower alkyl-diarylsilyl such as methyl-diphenylsilyl, ethyl-diphenylsilyl, propyl-diphenylsilyl, and tert-butyl-diphenylsilyl, more preferably tri($C_1$-$C_4$)alkylsilyl group and $C_1$-$C_4$ alkyl-diphenylsilyl group, most preferably tert-butyl-dimethyl silyl group and tert-butyl-diphenylsilyl group; acyl groups such as aliphatic acyl groups, aromatic acyl groups derived from carboxylic acids, sulfonic acids and carbamic acids and aliphatic acyl groups substituted with aromatic groups.

Examples of "aliphatic acyl groups" include lower alkanoyl groups which may have one or more suitable substituent such as carboxy (e.g., formyl, acetyl, propionyl, butyryl, isobutyryl, valeryl, isovaleryl, pivaloyl, hexanoyl, carboxyacetyl, carboxypropionyl, carboxybutyryl, carboxyhexanoyl), cyclo(lower)alkoxy-(lower)alkanoyl groups which may have one or more suitable substituent such as lower alkyl (e.g., cyclopropyloxyacetyl, cyclobutyloxypropionyl, cycloheptyloxybutyryl, menthyloxyacetyl, menthyloxypropionyl, menthyloxybutyryl, menthyloxypentanoyl, menthyloxyhexanoyl), camphorsulfonyl group; lower alkylcarbamoyl groups having one or more suitable substituent such as carboxy or protected carboxy group, as exemplified by carboxy(lower)-alkylcarbamoyl groups (e.g., carboxymethylcarbamoyl, carboxyethylcarbamoyl, carboxypropylcarbamoyl, carboxybutylcarbamoyl, carboxypentylcarbamoyl, carboxyhexylcarbamoyl) or protected carboxy(lower)alkylcarbamoyl groups such as tri(lower)alkylsilyl(lower)-alkoxycarbonyl(lower)alkylcarbamoyl groups (e.g., trimethylsilylmethoxycarbonylethylcarbamoyl, trimethylsilylethoxycarbonylpropylcarbamoyl, triethylsilylethoxycarbonylpropylcarbamoyl, t-butyldimethylsilylethoxycarbonylpropylcarbamoyl, and trimethylsilylpropoxycarboylbutylcarbamoyl).

Examples of the "aromatic acyl group" include aroyl groups which may have one or more suitable substituent such as nitro (e.g., benzoyl, toluoyl, xyloyl, naphthoyl, nitrobenzoyl, dinitrobenzoyl, and nitronaphthoyl), and arenesulfonyl groups which may have one or more suitable substituent such as halogen (e.g., benzenesulfonyl, toluenesulfonyl, xylenesulfonyl, naphthalene-sulfonyl, fluorobenzenesulfonyl, chlorobenzensulfonyl, bromo-benzenesulfonyl, iodobenzenesulfonyl).

Examples of the aliphatic acyl group substituted with aromatic group include ar(lower) alkanoyl groups which may have one or more suitable substituent such as lower alkoxy and trihalo(lower)alkyl (e.g., phenylacetyl, phenylpropionyl, phenylbutyryl, 2-trifluoromethyl-2-methoxy-2-phenylacetyl,2-ethyl-2-trifluoromethyl-2-phenylacetyl, 2-trifluoromethyl-2-propoxy-2-phenylacetyl).

Among the above-mentioned acyl groups, further preferable acyl groups are $C_1$ - $C_4$ alkanoyl groups which may have carboxy, cyclo($C_5$ - $C_6$)alkyloxy($C_1$ - $C_4$) alkanoyl groups having 2 ($C_1$ - $C_4$)alkyls at the cycloalkyl moiety, camphorsulfonyl group, carboxy($C_1$ - $C_4$)alkylcarbamoyl groups, tri($C_1$ - $C_4$)alkylsilyl($C_1$ - $C_4$)alkoxycarbonyl($C_1$ - $C_4$)alkylcarbamoyl groups, benzoyl groups which may have 1 or 2 nitro group, benzensulfonyl groups having halogen, phenyl($C_1$ - $C_4$) alkanoyl groups having $C_1$ - $C_4$ alkoxy and trihalo($C_1$ - $C_4$) alkyl, and thereamong, most preferable are acetyl, carboxypropionyl, methyloxyacetyl, camphorsulfonyl, benzoyl, nitrobenzoyl, dinitrobenzoyl, iodobenzenesulfonyl and 2-trifluoromethyl-2-methoxy-2-phenylacetyl.

Preferable examples of the "heterocyclic group having a nitrogen atom, sulfur atom or oxygen atom comprising a 5- or 6-membered ring" include pyrrolyl group, tetrahydrofuryl group, and others.

As the "pharmaceutically acceptable salt" of the tricyclo compound (I), there are included non-toxic pharmaceutically acceptable salts, namely salts with inorganic or organic bases such as alkali metal salts (e.g., sodium, potassium salts), alkaline earth metal salts (e.g., calcium, magnesium salts), and ammonium salts, amine salts (e.g., triethylamine, N-benzyl-N-methylamine salts).

In the tricyclo compound (I), there may exist a pair or more of steric isomers such as conformers, optical isomers and geometrical isomers, due to an asymmetric carbon atom and double bond, and such conformers or isomers are also included within the scope of the present invention.

Among the tricyclo compounds (I) to be used in the present invention, the compound represented by the following structural formula is the most preferred.

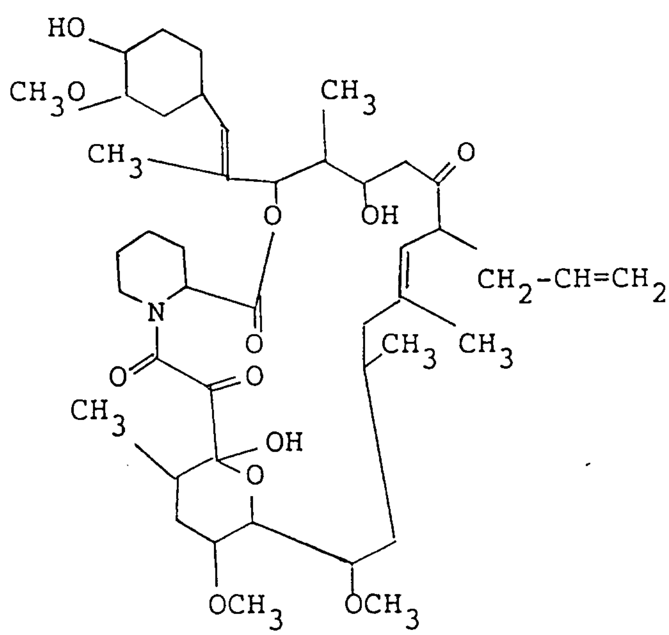

(hereinafter called FK-506).

The tricyclo compound (I) or a salt thereof can be obtained by the process described in JP-A-61-148181 and EP-A-0323042. Particularly, FK-506 and an analogue thereof can be produced by fermentation of Streptomyces tsukubaensis No. 9993(FERM BP-927), and Streptomyces hygroscopicus subsp. yakushimaensis (FERM BP-928), respectively.

In the present specification, the term hair revitalizing effect or hair revitalizing action means epilation prevention, hair germination, and a promotion of hair generation and hair growth.

Preparation formation

Next, the preparation of the tricyclo compound (I) for application as a hair revitalizing material is described.

The Aricyclo compound used according to the present invention is used in the form of a mixture in which, in addition to the tricyclo compound (I), acceptable additives and other drugs conventionally used as hair revitalizing materials are added. Examples of these additives and drugs include antibacterial agents such as hinokitiol, hexachlorophen, phenol, benzalkonium chloride, cetylpyridinium chloride, undecylenic acid, trichlorocarbanilide and bithionol, antiphlogistic agents or refrigerants such as glycyrrhizinic acid and derivatives thereof (e.g., ammonium salt), allantoin, menthol, skin activators such as pantothenic acid and derivatives thereof, pantothenol and derivatives thereof, saponin, circulation promoters such as Swelchnogen, vitamin E nicotinic acid ester, cefratin, skin peripheral vasodilators such as capronium chloride, vitamine B6 hydrochloride, acetylcholin derivatives, keratolytic agents such as salicyclic acid, resorcin, drugs such as zinc and compounds thereof, galenical extracts such as of capsicum tincture, licorice, lithospermum root, runner, ginseng, animal and vegetable oils such as olive oil, macadamia nut, squalane, hydrocarbon oils as represented by fluid paraffin, ester oils such as isopropyl myristate, 2-ethylhexylpalmitate, waxes such as beeswax, carunauba wax, oils such as higher fatty acids, higher alcohols, lactic acid and derivatives thereof such as alkyl ester, etc., polyhydric alcohols such as polyethylene glycol, glycerine, sorbitol, humectants such as mucopolysaccharides, pyrrolidone carboxylic acid salts, thickeners such as hydroxypropylmethyl cellulose, carboxyvinyl polymer, gelatin, gum arabic, polyvinyl alcohol, lower alcohols such as ethanol, water, vitamins, hormones, amino acids, surfactants, solubilizing agents, antioxidants, UV-ray absorbers, perfumes, and dyes, and these may be used alone or as a mixture of two or more thereof.

The dosage form of the hair revitalizing agent can be any desired form, provided that it can be externally applied. For example, external drug creams, such as lotions, liniments, milky lotions, external semi-solid preparations such as ointments, pasta, jelly, sprays, and hair shampoos and hair rinses can be included.

In the hair revitalizing agent the amount formulated of the tricyclo compound (I), which is the active ingredient, is not particularly limited, but may be formulated within the range of, for example, from 0.0001 to 60% by weight (hereinafter % represents % by weight), preferably from 0.001 to 10%, most preferably from 0.005 to 0.8%.

Administration form

The hair revitalizing agent is administered by a percutaneous administration or by spraying onto the skin. The dose of the hair revitalizing agent varies depending on the age, individual differences, and the condition of the disease, and cannot be clearly defined, but in general, the percutaneous dose of the tricyclo compound (I) for a human being is preferably 0.00001 to 1000 mg, more preferably 0.0001 to 500 mg, per day. The above-mentioned dose can be administered once or in 2 to 4 divided portions per day.

Hair revitalizing test example

(1) Hair generation test with mouse I

To determine the hair revitalizing effect a hair generation test using a mouse was conducted. The back of a C3H/HeNCrj male mouse at the resting stage was depilated by a razor, and the three kinds of samples shown in Table 1 were each coated thereon in an amount of 0.12 ml, once per day, and the conversion from the resting stage to the growing stage was observed.

In the groups to which the test drugs of Samples 1 and 2 were administered, a complete conversion from the resting stage to the growing stage was observed over the whole region of the depilated portion, with all of the examples, after an elapse of about 1 week, whereby a hair generation was rapidly commenced. In the group administered with Sample 3 used as Control, after the elapse of about one week, only a conversion to the growing stage was observed locally at the depilated portion, in only one example, with the remainder all at the resisting stage.

Table 1

| Group | Composition of test solution |
|---|---|
| 1 | 50% ethanolic solution containing 0.1% FK-506 |
| 2 | 50% ethanolic solution containing 1% FK-506 |
| 3 (Control) | 50% ethanol |

(2) Hair generation test with mouse II

The back of a C3H/HeNCrj strain male mouse at the resting stage was depilated by a razor, and three kinds of samples shown in Table 2 were each coated thereon in an amount of 0.1 ml, once per day. The effect was judged from the hair generation area ratio, calculated on the basis of photographs.

Table 2

| Group | Composition of test solution |
|---|---|
| 4 | 70% ethanolic solution containing 0.1% FK-506 |
| 5 | 70% ethanolic solution containing 1% α-tocopherol acetate |
| 6 (Control) | 70% ethanol |

Results

Table 3 shows the hair generation area ratio and determination 2 weeks after the coating.

Table 3

| Sample | Hair generation area ratio (%)* | Determination |
|---|---|---|
| 4 | 75.3 ± 8.5 | Very strong effect |
| 5 | 5.9 ± 3.2 | Weak effect |
| 6 | 0.0 ± 0.0 | No effect |

* Mean ± S.E.

As apparent from the above results, the tricyclo compound (I) used according to the invention has a remarkable effect as a hair revitalizing agent.

[Examples]

Referring now to Examples, the preparation method of the hair revitalizing agent is described in detail. In the Examples, % indicates % by weight.

Example 1

A lotion comprising the composition shown below was prepared.

| | (%) |
|---|---|
| 95% Ethanol | 80.0 |
| FK-506 | 10.0 |
| α-Tocopherol acetate | 0.01 |
| Ethylene oxide (40 mole) adducts of hardened castor oil | 0.5 |
| Purified water | 9.0 |
| perfume and dye | q.s. |

Into 95% ethanol were added FK-506, α-tocopherol acetate, ethylene oxide (40 mole) adducts of hardened castor oil, perfume, and a dye, and the mixture was stirred and dissolved, followed by an addition of purified water, to obtain a transparent liquid lotion.

The lotion was coated once or twice per day, in an amount of 5 ml each time, at a site having marked baldness or alopecia.

Example 2

A lotion comprising the composition shown below was prepared. (%)

| | |
|---|---|
| 95% Ethanol | 80.0 |
| FK-506 | 0.005 |
| Hinokitiol | 0.01 |
| Ethylene oxide (40 mole) adducts of hardened castor oil | 0.5 |
| Purified water | 19.0 |
| Perfume and dye | q.s. |

Into 95% ethanol were added FK-506, hinokitiol, ethylene oxide (40 mole) adducts of hardened castor oil, perfume, and a dye, and the mixture was stirred and dissolved, followed by an addition of purified water, to obtain a transparent liquid lotion.

The lotion was coated by spraying once to 4 times per day.

Example 3

An emulsion was prepared from A phase and B phase having the following compositions.

| (A phase) | (%) |
|---|---|
| Whale wax | 0.5 |
| Cetanol | 2.0 |
| Petrolatum | 5.0 |
| Squalane | 10.0 |
| Polyoxyethylene (10 mole) monostearate | 2.0 |
| Sorbitane monooleate | 1.0 |
| FK-506 | 0.01 |

| (B phase) | (%) |
|---|---|
| Glycerine | 10.0 |
| Purified water | 69.0 |
| Perfume, dye, and preservative | q.s. |

The A phase and the B phase were respectively heated and melted and maintained at 80 °C, both phases were mixed to be emulsified, and were cooled under stirring to normal temperature to obtain an emulsion. The emulsion was coated by spraying once to four times per day.

Example 4

A cream was prepared from A phase and B phase having the following compositions.

| (A phase) | (%) |
|---|---|
| Fluid paraffin | 5.0 |
| Cetostearyl alcohol | 5.5 |
| Petrolatum | 5.5 |
| Glycerine monostearate | 3.0 |
| Polyoxyethylene (20 mole) 2-octyldodecyl ether | 3.0 |
| Propylparaben | 0.3 |

| (B phase) | (%) |
|---|---|
| FK-506 | 0.8 |
| Glycerine | 7.0 |
| Dipropylene glycol | 20.0 |
| Polyethylene glycol 4000 | 5.0 |
| Sodium hexametaphosphate | 0.005 |
| Purified water | 44.895 |

The A phase is heated and melted, and maintained at 70 °C, the B phase was added into the A phase followed by stirring, and the obtained emulsion was cooled to obtain a cream.

The cream was coated on the skin once to 4 times per day.

Example 5

A hair liquid comprising the composition shown below was prepared.

| | (%) |
|---|---|
| Polyoxyethylene butyl ether | 20.0 |
| Ethanol | 50.0 |
| FK-506 | 0.001 |
| Propylene glycol | 5.0 |
| Polyoxyethylene hardened castol oil derivative (ethylene oxide 80 mole adducts) | 0.4 |
| Perfume | q.s. |
| Purified water | q.s. |

Into ethanol were added polyoxypropylene butyl ether, propylene glycol, polyoxyethylene hardened castor oil, FK-506, and perfume, which were mixed under stirring, and to the mixture was added purified water, to obtain a hair liquid.

Example 6

A hair shampoo comprising the composition shown below was prepared.

| | (%) |
|---|---|
| Sodium laurylsulfate | 5.0 |
| Triethanolamine laurylsulfate | 5.0 |
| Betaine lauryldimethylaminoacetate | 6.0 |
| Ethylene glycol distearate | 2.0 |
| Polyethylene glycol | 5.0 |
| FK-506 | 5.0 |
| Ethanol | 2.0 |
| Perfume | 0.3 |
| Purified water | 69.7 |

Into 69.7 g of purified water were added 5.0 g of sodium laurylsulfate, 5.0 g of triethanolamine laurylsulfate, 6.0 g of betaine lauryldimethylaminoacetate, then a mixture obtained by adding 5.0 g of FK-506, 5.0 g of polyethylene glycol, and 2.0 g of ethylene glycol distearate to 2.0 g of ethanol, followed by stirring, and 0.3 g of perfume, were successively added, and the mixture was heated then cooled to obtain a hair shampoo. The hair shampoo was used on the scalp once or twice per day.

**Claims**

**1.** Use of a compound represented by the formula shown below, or a pharmaceutically acceptable salt thereof:

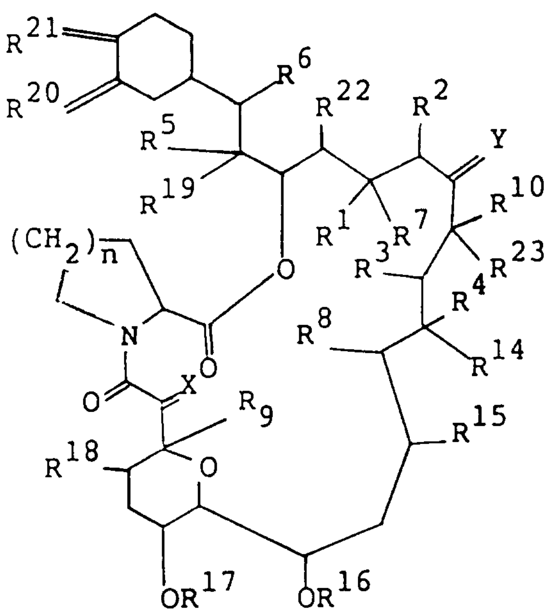

(I)

(wherein each adjoining pair of $R^1$ and $R^2$, $R^3$ and $R^4$, $R^5$ and $R^6$ independently,

a) represents two adjoining hydrogen atoms, or

b) forms another bond with a carbon atom to which it is bonded, and in addition thereto, $R^2$ may be an alkyl group;

$R^7$ represents a hydrogen atom, a hydroxy group, a protected hydroxy group, or an alkyloxy group, or represents an oxo group together with $R^1$;

$R^8$ and $R^9$ independently represent a hydrogen atom, or a hydroxy group;

$R^{10}$ represents a hydrogen atom, an alkyl group, an alkyl group substituted with 1 or more hydroxy groups, an alkenyl group, an alkenyl group substituted with 1 or more hydroxy groups, or an alkyl group substituted with an oxo group;

X represents an oxo group, (hydrogen atom, hydroxy group), (hydrogen atom, hydrogen atom) or a group represented by $-CH_2O-$;

Y represents an oxo group, (hydrogen atom, hydroxy group), (hydrogen atom, hydrogen atom), or a group represented by the formula $N-NR^{11}R^{12}$ or $N-OR^{13}$;

$R^{11}$ and $R^{12}$ independently represent a hydrogen atom, an alkyl group, an aryl group or a tosyl group;

$R^{13}$, $R^{14}$, $R^{15}$, $R^{16}$, $R^{17}$, $R^{18}$, $R^{19}$, $R^{22}$ and $R^{23}$ independently represent a hydrogen atom or an alkyl group;

$R^{20}$ and $R^{21}$ independently represent an oxo group, or may be each independently ($R_a^{20}$ , hydrogen atom) and ($R_a^{21}$ , hydrogen atom); $R_a^{20}$ and $R_a^{21}$ independently represent a hydroxy group, an alkyloxy group or a group represented by the formula $OCH_2OCH_2CH_2OCH_3$ , or $R^{21}$ represents a protected hydroxy group, and further, $R_a^{20}$ and $R_a^{21}$ taken together form an oxygen atom in an epoxy ring;

n represents 1, 2 or 3, and

in addition to the above meanings, Y, $R^{10}$ and $R^{23}$ together with the carbon atoms to which they are bonded may represent a heterocyclic group containing nitrogen atom, sulfur atom or oxygen atom comprising a saturated or unsaturated 5- or 6-membered ring, and the heterocyclic group may be substituted with 1 or more group selected from alkyl groups, hydroxy group, alkyl groups substituted with 1 or more hydroxy group, alkyloxy groups, benzyl group and the group represented by $-CH_2Se(C_6H_5)$,

for the manufacture of a medicament for treating male pattern alopecia or alopecia senilis.

**2.** Use according to claim 1; wherein the compound (I) is FK-506.

**3.** Use according to claim 1 or 2, wherein the compound (I) is applied externally.

**Patentansprüche**

**1.** Verwendung einer Verbindung, die die durch unten gezeigte Formel dargestellt wird, oder eines pharmazeutisch verträglichen Salzes davon:

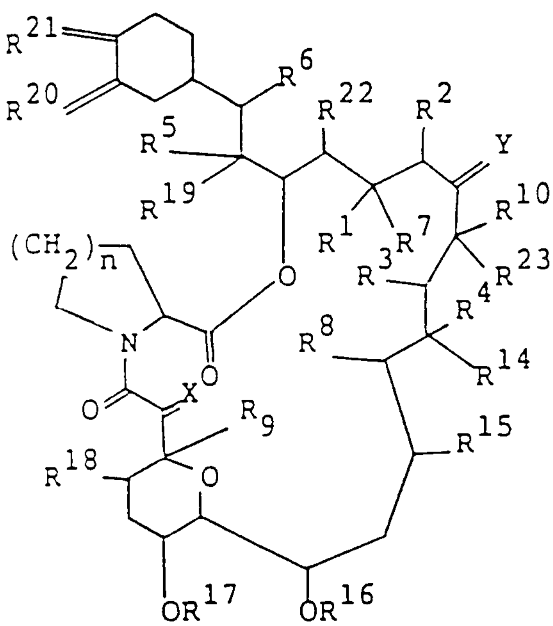

(I)

worin jedes nebeneinanderliegende Paar von $R^1$ und $R^2$, $R^3$ und $R^4$, $R^5$ und $R^6$ unabhängig voneinander,

a) zwei nebeneinanderliegende Wasserstoffatome darstellt oder

b) eine weitere Bindung mit einem Kohlenstoffatom bildet, an die es gebunden ist, und zusätzlich dazu $R^2$ eine Alkylgruppe sein kann;

$R^7$ ein Wasserstoffatom, eine Hydroxygruppe, eine geschützte Hydroxygruppe oder eine Alkyloxygruppe darstellt oder zusammen mit $R^1$ eine Oxogruppe darstellt;

$R^8$ und $R^9$ unabhängig voneinander ein Wasserstoffatom oder eine Hydroxygruppe darstellen;

$R^{10}$ ein Wasserstoffatom, eine Alkylgruppe, eine Alkylgruppe substituiert mit 1 oder mehr Hydroxygruppen, eine Alkenylgruppe, eine Alkenylgruppe substituiert mit 1 oder mehr Hydroxygruppen oder eine Alkylgruppe substituiert mit einer Oxogruppe darstellt;

X eine Oxogruppe (Wasserstoffatom, Hydroxygruppe), (Wasserstoffatom, Wasserstoffatom) oder eine durch $-CH_2O-$ dargestellte Gruppe darstellt;

Y eine Oxogruppe (Wasserstoffatom, Hydroxygruppe), (Wasserstoffatom, Wasserstoffatom), oder eine durch die Formel $N-NR^{11}R^{12}$ oder $N-OR^{13}$ dargestellte Gruppe darstellt;

$R^{11}$ und $R^{12}$ unabhängig voneinander ein Wasserstoffatom, eine Alkylgruppe, eine Arylgruppe oder eine Tosylgruppe darstellen;

$R^{13}$, $R^{14}$, $R^{15}$, $R^{16}$, $R^{17}$, $R^{18}$, $R^{19}$, $R^{22}$ und $R^{23}$ unabhängig voneinander ein Wasserstoffatom oder eine Alkylgruppe darstellen;

$R^{20}$ und $R^{21}$ unabhängig voneinander eine Oxogruppe darstellen oder jede unabhängig voneinander ($R^{20}{}_a$, Wasserstoffatom) und ($R^{21}{}_a$, Wasserstoffatom) sein können; $R^{20}{}_a$ und $R^{21}{}_a$ unabhängig voneinander eine Hydroxygruppe, eine Alkyloxygruppe oder eine durch die Formel $OCH_2OCH_2CH_2OCH_3$ dargestellte Gruppe darstellen oder $R^{21}{}_a$ eine geschützte Hydroxygruppe darstellt und weiter $R^{20}{}_a$ und $R^{21}{}_a$ zusammengenommen ein Sauerstoffatom in einem Epoxyring bilden;

n 1, 2 oder 3 darstellt und

zusätzlich zu den obigen Bedeutungen Y, $R^{10}$ und $R^{23}$ zusammen mit den Kohlenstoffatomen, an die sie gebunden sind, eine heterocyclische, einen gesättigten oder ungesättigten 5- oder 6-gliedrigen Ring umfassende Gruppe darstellen können, die ein Stickstoffatom, Schwefelatom oder Sauerstoffatom enthält, und die heterocyclische Gruppe mit einer oder mehr Gruppen, ausgewählt aus Alkylgruppen, Hydroxygruppe, Alkylgruppen substituiert mit 1 oder mehr Hydroxygruppe, Alkyloxygruppen, Benzylgruppe und der durch $-CH_2Se(C_6H_5)$ dargestellten Gruppe substituiert sein kann,

zur Herstellung eines Medikamentes zur Behandlung von Calvities oder Altersalopezie.

**2.** Verwendung nach Anspruch 1, worin die Verbindung (I) FK-506 ist.

**3.** Verwendung nach Anspruch 1 oder 2, worin die Verbindung (I) äußerlich angewendet wird.

### Revendications

**1.** Utilisation d'un composé représenté par la formule montrée ci-dessous, ou un sel pharmaceutiquement acceptable de celui-ci:

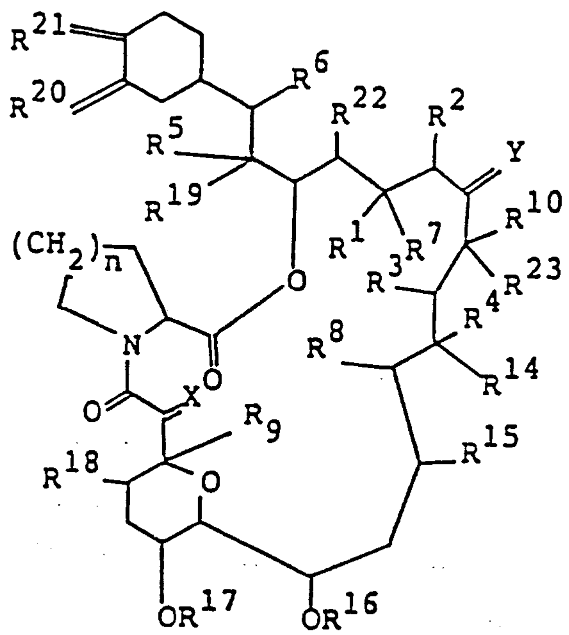

(I)

(où chaque paire contiguë de $R^1$ et $R^2$, $R^3$ et $R^4$, $R^5$ et $R^6$ indépendamment,

a) représente deux atomes d'hydrogène contigus, ou

b) forme une autre liaison avec un atome de carbone auquel elle est liée, et en plus de cela, $R^2$ peut être un groupe alkyle;

$R^7$ représente un atome d'hydrogène, un groupe hydroxy, un groupe hydroxy protégé, ou un groupe alkyloxy, ou représente un groupe oxo ensemble avec $R^1$;

$R^8$ et $R^9$ indépendamment représentent un atome d'hydrogène, ou un groupe hydroxy;

$R^{10}$ représente un atome d'hydrogène, un groupe alkyle, un groupe alkyle substitué avec 1 ou plusieurs groupes hydroxy, un groupe alcényle, un groupe alcényle substitué avec un ou plusieurs groupes hydroxy, ou un groupe alkyle substitué avec un groupe oxo;

X représente un groupe oxo, (atome d'hydrogène, groupe hydroxy), (atome d'hydrogène, atome d'hydrogène) ou un groupe représenté par $-CH_2O-$;

Y représente un groupe oxo, (atome d'hydrogène, groupe hydroxy), (atome d'hydrogène, atome d'hydrogène), ou un groupe représenté par la formule $N-NR^{11}R^{12}$ ou $N-OR^{13}$;

$R^{11}$ et $R^{12}$ indépendamment représentent un atome d'hydrogène, un groupe alkyle, un groupe aryle ou un groupe tosyle;

$R^{13}$, $R^{14}$, $R^{15}$, $R^{16}$, $R^{17}$, $R^{18}$, $R^{19}$, $R^{22}$ et $R^{23}$ indépendamment représentent un atome d'hydrogène ou un groupe alkyle;

$R^{20}$ et $R^{21}$ indépendamment représentent un groupe oxo, ou peuvent être chacun indépendamment ($R_a^{20}$ , atome d'hydrogène) et ($R_a^{21}$ , atome d'hydrogène); $R_a^{20}$ et $R_a^{21}$ indépendamment représentent un groupe hydroxy, un groupe alkyloxy ou un groupe représenté par la formule $OCH_2OCH_2CH_2OCH_3$, ou $R_a^{21}$ représente un groupe hydroxy protégé, et en outre, $R_a^{20}$ et $R_a^{21}$ pris ensemble forment un atome d'oxygène dans un cycle époxy;

n représente 1, 2, ou 3, et

en plus des significations précédentes, Y, $R^{10}$, et $R^{23}$ ensemble avec les atomes de carbone auxquels ils sont liés peuvent représenter un groupe hétérocyclique contenant un atome d'azote, un atome de soufre ou un atome d'oxygène comprenant un cycle à 5 ou 6 membres saturé ou insaturé, et le groupe hétérocyclique peut être substitué avec un ou plusieurs groupes choisis parmi des groupes alkyles, un groupe hydroxy, des groupes alkyles substitués avec un ou plusieurs groupes hydroxy, des groupes alkyloxy, un groupe benzyle et le groupe représenté par $-CH_2Se(C_6H_5)$, pour la fabrication d'un médicament pour traiter l'alopécie masculine ou l'alopécie sénile.

**2.** Utilisation selon la revendication 1, où le composé (I) est FK-506.

**3.** Utilisation selon la revendication 1 ou 2, où le composé (I) est appliqué de manière externe.